# EUROPEAN PATENT SPECIFICATION

(11) **EP 2 004 201 B1**
(45) Date of publication and mention of the grant of the patent: **08.08.2018**
(21) Application number: 07754349.4
(22) Date of filing: 27.03.2007
(51) Int. Cl.: A23K 40/30, A23K 10/18, A23K 20/174, A23K 20/163, A23K 20/20, A23K 50/40, A23L 33/135, A23L 33/15, A23L 33/16, A23L 33/21, A61K 38/00, A61K 45/06, A61K 9/50, A61K 31/01, A61K 31/145, A61K 31/375, A23K 20/22, A61K 36/064, A61K 31/355, A61K 35/66

(54) **DIETARY SUPPLEMENTS CONTAINING PROBIOTICS**
ERNÄHRUNGSERGÄNZUNGEN MIT PROBIOTIKA
SUPPLÉMENTS ALIMENTAIRES CONTENANT DES PROBIOTIQUES

(30) Priority: 29.03.2006 US 787257 P
(43) Date of publication of application: 24.12.2008
(73) Proprietor: Nestec S.A., 1800 Vevey (CH)
(72) Inventor: CZARNECKI-MAULDEN, Gail, Glen Carbon, Illinois 62034 (US); FILIPI, Ivan, St. Louis, Missouri 63128 (US); CAVADINI, Christoph, 1804 Corsier-sur-Vevey (CH)
(74) Representative: Rupp, Christian
(86) International application number: PCT/US2007/007817
(87) International publication number: WO 2007/126990

(56) References cited:
- EP-A1- 1 344 458
- WO-A1-01/90311
- WO-A1-2005/030229
- WO-A2-2006/110406
- GB-A- 2 334 443
- JP-A- 2005 508 647
- US-A- 5 200 218
- US-A- 5 968 569
- US-A1- 2003 152 629
- US-B1- 6 254 886
- US-B1- 6 660 319
- JALIL BENYACOUB ET AL: "Nutritional Immunology Supplementation of Food with Enterococcus faecium (SF68) Stimulates Immune Functions in Young Dogs 1", THE JOURNAL OF NUTRITION, 7 January 2003 (2003-01-07), pages 1158-1162, XP055124592,
- Taras Kuzio ET AL: "Performance, diarrhea incidence, and occurrence of Escherichia coli virulence genes during long-term administration of a probiotic Enterococcus faecium strain to sows and piglets", Journal of animal science, 1 March 2006 (2006-03-01), pages 608-617, XP055124608, DOI: 10.1080/13523270701194813 Retrieved from the Internet: URL:http://www.journalofanimalscience.org/ content/84/3/608.full.pdf#page=1&view=FitH [retrieved on 2014-06-23]
- "Opinion of the Scientific Panel on Additives and Products or Substances used in Animal Feed on a request from the Commission on the safety of the product Cylactin for dogs and cats (Question N O EFSA Q-2004-006)", The EFSA Journal, 2 December 2004 (2004-12-02), pages 1-7, XP055125085, Retrieved from the Internet: URL:http://www.efsa.europa.eu/en/efsajourn al/doc/138.pdf [retrieved on 2014-06-25]
- DATABASE PUBMED [Online] ROBERFROID M.B.: 'Probiotics and Probiotics: are they functional foods?', XP008135061 Retrieved from STN Database accession no. (10837317) & THE AMERICAN JOURNAL OF NUTRITION vol. 71, no. 6, SUPPL., June 2000, pages 1682S - 1687S
- Anonymous: "GNPD - Lite Cat Food", , 1 August 1999 (1999-08-01), XP055376839, Retrieved from the Internet: URL:http://www.gnpd.com/sinatra/recordpage /10061952/from_search/nn0d0v8kay/?page=4 [retrieved on 2017-05-30]
- Anonymous: "GNPD - Adult Cat Food", , 1 October 2001 (2001-10-01), XP055376842, Retrieved from the Internet: URL:http://www.gnpd.com/sinatra/recordpage /10094464/from_search/nn0d0v8kay/?page=4 [retrieved on 2017-05-30]
- Anonymous: "GNPD - Lite Cat Food Extension", , 1 December 2003 (2003-12-01), XP055376844, Retrieved from the Internet: URL:http://www.gnpd.com/sinatra/recordpage /10156768/from_search/nn0d0v8kay/?page=4 [retrieved on 2017-05-30]

## Description

### BACKGROUND OF THE INVENTION

### Field of the Invention

The invention relates generally to dietary supplements and particularly to dietary supplements containing probiotics.

### Description of the Related Art

The intestinal tract plays a critical role in animal health and wellness. To help fulfill this role, the intestinal tract contains various microorganisms that comprise a healthy gastrointestinal microflora under normal conditions. The microflora confers many benefits to the animal, e.g., the production of fatty acids that fuel the cells that line the gastrointestinal lumen, the synthesis of vitamins, and the synthesis of enzymes that aid in the breakdown and digestion of food. In addition, the microflora aids the immune system in host protection from disease. For example, microflora are known to inhibit the attachment to and colonization of potential pathogens within the gastrointestinal tract and to stimulate the production of cytokines and immunoglobulins.

Unfortunately, disruption of the normal balance of microflora can result in opportunistic infection of the gastrointestinal tract and can facilitate additional complications such as diarrhea and dehydration. The normal microflora balance can be disrupted through a variety of means, e.g., stress, advanced age, travel, consumption of contaminated food or water, antibiotic therapy, and the like. One method for preventing or treating such undesirable disruption involves prophylactically administering probiotics to an animal to prevent the disruption or to therapeutically administering probiotics to an animal to restore the normal microflora balance and facilitate recovery from the resulting undesirable complications caused by the disruption.

Probiotics and their benefits for animal health are well known to skilled artisans. Probiotics are live microorganisms that have a beneficial effect in the prevention and treatment of specific medical conditions when ingested. Probiotics are believed to exert biological effects through a phenomenon known as colonization resistance. Probiotics facilitate a process whereby the indigenous anaerobic flora limits the concentration of potentially harmful (mostly aerobic) bacteria in the digestive tract. Other modes of action, such as supplying enzymes or influencing enzyme activity in the gastrointestinal tract, may also account for some of the other functions that have been attributed to probiotics. Probiotics are known to enhance intestinal function, stimulate the immune system, reduce inflammation, and diminish the population of harmful microorganisms in the gastrointestinal tract. The products Lite Cat Food of Innova, Precise Plus Adult Cat Food of Precise Plus and Blue lite Cat Food of Blue Buffalo found in the GNPD database comprise non-encapsulated probiotics and at least one of animal digest, dried brewers yeast, vitamin C; vitamin E, beta carotene, zinc proteinate, manganese proteinate, ferrous sulfate, copper proteinate, calcium iodate, and sodium selenite, but not all of these compounds.

While probiotics are generally useful for promoting the health of an animal, they are often difficult to store, handle, and administer to the animal. Probiotics may be unstable under normal environmental conditions and require special handling, e.g., refrigeration, freeze drying, or other means to prolong probiotic life. Similarly, probiotics are often unpalatable to the animal consuming them. Often, the palatability must be disguised or enhanced using other compounds or compositions. Further, it may be beneficial to supplement the immune system, particularly in the gastrointestinal tract, using ingredients in combination with the probiotics to obtain maximum benefit from probiotic administration. There is, therefore, a need for new compositions containing probiotics, particularly in the form of dietary supplements, that have enhanced palatability compared to the probiotics alone, contain ingredients that enhance the immune system to augment the action of the probiotics, and/or have an extended life compared to the probiotics when they are not in the compositions.

### SUMMARY OF THE INVENTION

It is, therefore, an object of the present invention to provide dietary supplements containing probiotics.

It is another object of the present invention to provide dietary supplements containing microencapsulated probiotics.

It is a further object of the present invention to provide dietary supplements containing probiotics that have enhanced palatability compared to the probiotics alone.

It is another object of the present invention to provide food or other compositions that have enhanced palatability when supplemented with the dietary supplement of the present invention.

It is another object of the present invention to provide dietary supplements containing probiotics and other ingredients that enhance the immune system to augment the action of the probiotics.

It is another object of the present invention to provide dietary supplements containing probiotics that have an extended life compared to the probiotics when they are not in the dietary supplements.

It is a further object of the invention to provide articles of manufacture in the form of kits that contain combinations of the dietary supplements of the present invention, food compositions, compounds, and devices, particularly combinations useful for promoting gastrointestinal health and the health or wellness of an animal.

One or more of these and other objects are achieved using novel dietary supplements that contain probiotics and ingredients that increase palatability, enhance immunity, and/or extend the life of the probiotics in the supplements of compositions containing the supplements. Generally, the supplements comprise at least one probiotic and at least one of animal digest, dried brewers yeast, vitamin C; vitamin E, beta carotene, zinc proteinate, manganese proteinate, ferrous sulfate, copper proteinate, calcium iodate, and sodium selenite. The supplement contains the probiotics and other ingredients in amounts sufficient to administer beneficial amounts of probiotics to the animal and to enhance palatability, enhance the immune system, and/or extend probiotic life.

Other and further objects, features, and advantages of the present invention will be readily apparent to those skilled in the art.

### DETAILED DESCRIPTION OF THE INVENTION

### Definitions

The term "animal" means a human or other animal, including avian, bovine, canine, equine, feline, hicrine, murine, ovine, and porcine animals, that can benefit from the administration of probiotics.

The term "dietary supplement" means a product that is intended to be ingested in addition to the normal diet of an animal.

The term "probiotic" means any microorganism that exerts a beneficial effect on the host animal such as increased health or resistance to disease. Probiotics can exhibit one or more of the following non-limiting characteristics: non-pathogenic or non-toxic to the host; are present as viable cells, preferably in large numbers; capable of survival, metabolism, and persistence in the gut environment (e.g., resistance to low pH and gastrointestinal acids and secretions); adherence to epithelial cells, particularly the epithelial cells of the gastrointestinal tract; microbicidal or microbistatic activity or effect toward pathogenic bacteria; anticarcinogenic activity; immune modulation activity, particularly immune enhancement; modulatory activity toward the endogenous flora; enhanced urogenital tract health; antiseptic activity in or around wounds and enhanced would healing; reduction in diarrhea; reduction in allergic reactions; reduction in neonatal necrotizing enterocolitis; reduction in inflammatory bowel disease; and reduction in intestinal permeability.

The term "viable" means alive and capable of reproduction or colonization.

The term "room temperature" means about 20°C to about 25°C.

The term "elevated temperature" means a temperature occurring during storage and distribution during hot season, having typical range of 30°C to 60°C.

The term "in conjunction" means that a dietary supplement, food composition, anti-diarrhea agent, or other compound or composition of the present invention are administered to an animal (1) together in a food composition or (2) separately at the same or different frequency using the same or different administration routes at about the same time or periodically. "Periodically" means that the agent is administered on a dosage schedule acceptable for a specific agent and that the food is fed to an animal routinely as appropriate for the particular animal. "About the same time" generally means that the food and agent are administered at the same time or within about 72 hours of each other. "In conjunction" specifically includes administration schemes wherein anti-diarrhea agent is administered for a prescribed period and the dietary supplements are administered indefinitely.

The term "anti-diarrhea agent" means any compound, composition, or drug useful for preventing or treating diarrhea.

The term "single package" means that the components of a kit are physically associated in or with one or more containers and considered a unit for manufacture, distribution, sale, or use. Containers include, but are not limited to, bags, boxes, bottles, shrink wrap packages, stapled or otherwise affixed components, or combinations thereof. A single package may be containers of individual food compositions physically associated such that they are considered a unit for manufacture, distribution, sale, or use.

The term "virtual package" means that the components of a kit are associated by directions on one or more physical or virtual kit components instructing the user how to obtain the other components, e.g., in a bag containing one component and directions instructing the user to go to a website, contact a recorded message, view a visual message, or contact a caregiver or instructor to obtain instructions on how to use the kit.

The term "health and wellness of an animal" means the complete physical, mental, and social well being of the animal, not merely the absence of disease or infirmity.

All percentages expressed herein are by weight of the composition on dry matter basis unless specifically stated otherwise. The term "dry matter basis" means that an ingredient's concentration in a composition is measured after any moisture in the composition is removed. All weights and concentrations for the compositions of the present invention are based on dry weight of a composition after all components and ingredients are admixed.

As used throughout, ranges are used for describing each and every value within the range. Any appropriate value within the range can be selected as the upper or lower value for the range.

The invention is not limited to the particular methodology, protocols, and reagents described herein because they may vary. Further, the terminology used herein is for the purpose of describing particular embodiments only and is not intended to limit the scope of the present invention. As used herein and in the appended claims, the singular forms "a," "an," and "the" include plural reference unless the context clearly dictates otherwise. Similarly, the words "comprise", "comprises", and "comprising" are to be interpreted inclusively rather than exclusively.

Unless defined otherwise, all technical and scientific terms and any acronyms used herein have the same meanings as commonly understood by one of ordinary skill in the art in the field of the invention. Although any compositions, methods, articles of manufacture, or other means or materials similar or equivalent to those described herein can be used in the practice of the present invention, the preferred compositions, methods, articles of manufacture, or other means or materials are described herein.

The present invention provides dietary supplements comprising at least one probiotic and animal digest, dried brewers yeast, vitamin C; vitamin E, beta carotene, zinc proteinate, manganese proteinate, ferrous sulfate, copper proteinate, calcium iodate, and sodium selenite. The invention is based upon the discovery that various combinations of probiotics and the above ingredients enhance the palatability of the probiotics and compositions containing the probiotics, enhance the immune system to augment the beneficial effects of the probiotics, and/or extend the life of the probiotics.

The probiotics can be present in the dietary supplements as an ingredient or additive. The probiotics can be prokaryotes, eukaryotes, or archaebacteria. Examples of suitable probiotics include yeasts such as Saccharomyces, Debaromyces, Candida, Pichia and Torulopsis, moulds such as Aspergillus, Rhizopus, Mucor, and Penicillium and Torulopsis and bacteria such as the genera Bifidobacterium, Bacteroides, Clostridium, Fusobacterium, Melissococcus, Propionibacterium, Streptococcus, Enterococcus, Lactococcus, Staphylococcus, Peptostrepococcus, Bacillus, Pediococcus, Micrococcus, Leuconostoc, Weissella, Aerococcus, Oenococcus and Lactobacillus. In preferred embodiments, the probiotics comprise at least one of any suitable strain or subspecies of Enterococcus, Streptococcus, Lactobacillus, Lactococcus, Bifidobacterium, or Saccharomyces. Enterococcus species include, without limitation, Enterococcus facecium, specifically Enterococcus faecium strain SF68 (NCIMB 10415), as well as other Enterococci Streptococcus species including, without limitation, Streptococcus faecium, Streptococcus thermophilus, and Streptococcus salivarus. Lactobacillus species include, without limitation, Lactobacillus acidophilus, Lactobacillus acidophilus NCC2628 (CNCM 1-2453), Lactobacillus acidophilus NCC2766, Lactobacillus acidophilus NCC2775, Lactobacillus brevis, Lactobacillus bulgaricus, Lactobacillus casei, Lactobacillus casei Imunitas, Lactobacillus casei Shirota, Lactobacillus cellobiosus, Lactobacillus crispatus, Lactobacillus curvatus, Lactobacillus delbrueckii, Lactobacillus fermentum, Lactobacillus gasseri, Lactobacillus helveticus, Lactobacillus johnsonii, Lactobacillus johnsonii NCC2774, Lactobacillus johnsonii NCC2767, Lactobacillus johnsonii NCC2822, Lactobacillus plantarum, Lactobacillus reuteri, Lactobacillus johnsonii LA1, Lactobacillus plantarum, Lactobacillus reuteri, Lactobacillus reuteri NCC2823, Lactobacillus reuteri NCC2581 (CNCM 1-2448), Lactobacillus reuteri NCC2592 (CNCM 1-2450), Lactobacillus reuteri NCC2603 (CNCM 1-2451), Lactobacillus reuteri NCC2613 (CNCM 1-2452), Lactobacillus rhamnosus Lactobacillus rhamnosus NCC2583 (CNCM 1-2449), Lactobacillus rhamnosus GG (ATCC 53103; Lactobacillus rhamnosus or Lactobacillus casei subspecies rhamnosus), Lactobacillus salivarius, Lactobacillus salivarius NCC2586, Lactobacillus paracasei ST11. Lactococcus species include, without limitation, Lactococcus lactis and Lactococcus plantarum. Bifidobacterium species include, without limitation, Bifidobacterium adolescentis, Bifidobacterium bifidum, Bifidobacterium animalis, Bifidobacterium thermophilum, Bifidobacterium breve, Bifidobacterium longum, Bifidobacterium pseudolongum, Bifidobacterium infantis, Bifidobacterium lactis, Bifidobacterium lactis Bb-12. Saccharomyces species include, without limitation, Saccharomyces boulardii (cerevisiae). In preferred embodiments, the dietary supplements comprise Enterococcus faecium (SF68).

The dietary supplements comprise probiotics in an amount suitable for enhancing the palatability of the probiotics and compositions containing the probiotics, enhancing the immune system to augment the beneficial effects of the probiotics, and/or extending the life of the probiotics. Generally, the dietary supplements comprise from about 1% to about 90% probiotics, preferably from about 1% to about 70% probiotics, most preferably from about 1% to about 50% probiotics.

The animal digest useful in the present invention can be any animal digest known to skilled artisans, e.g., any material that results from chemical and/or enzymatic hydrolysis of clean and undecomposed animal tissue. The dried brewers yeast useful in the present invention can be any dried brewers yeast known to skilled artisans, e.g., the dried, inactive agent that is a byproduct of the brewing industry. The animal digest and dried brewers yeast have been found to enhance the palatability of the dietary supplement and food or other compositions containing the dietary supplement. When present in the supplement, the animal digest comprises from about 10% to about 90% of the dietary supplement and the dried brewers yeast comprises from about 1% to about 30% of the dietary supplement.

The vitamin C, vitamin E, beta carotene, zinc proteinate, manganese proteinate, ferrous sulfate, copper proteinate, calcium iodate, and sodium selenite are compounds known to skilled artisans. These compounds have been found to enhance immunity and augment the action of the probiotics in the dietary supplement of the present invention. All of these compounds, the animal digest, and dried brewers yeast have been found in combination to extend the life of the probiotics compared to the probiotics when not in the compositions. When present in the supplement, the dietary supplement comprises from about 0.1% to about 10% vitamin C, from about 0.1% to about 10% vitamin E, from about 0.01% to about 3% beta carotene, from about 0.1% to about 5% zinc proteinate, from about 0.01% to about 3% manganese proteinate, from about 0.01% to about 3% ferrous sulfate, from about 0.01% to about 1% copper proteinate, from about 0.01% to about 1% calcium iodate, and from about 0.001% to about 0.1% sodium selenite. In a further embodiment, the dietary supplement further comprises from about 0.1% to about 2% taurine.

The dietary supplements can be prepared as a variety of formulations such as a powder, granule, pellet, or any other appropriate delivery form. In preferred embodiments, the dietary supplement formulation is powder containing microencapsulated probiotics within a biopolymer matrix. The powder dietary supplement can be sprinkled over or otherwise applied to and admixed with a food or other composition, particularly a pet food such as dog food or cat food. The powder dietary supplements can be specially formulated for consumption by a particular animal, such as companion animal. In one embodiment, the powder dietary supplement comprises a high concentration of probiotics such that the supplement can be administered to the animal in small amounts, or in the alternative, can be diluted before administration to an animal. A skilled practitioner can devise other routes of administration such as providing the dietary supplement alone or feeding it in, on, or with a pet treat.

The dietary supplements can be formulated for storage for prolonged periods at room temperature or, alternatively, can be refrigerated, freeze dried, or frozen. Preferably, the dietary supplement is stored at room temperature and the probiotics in the dietary supplement remain substantially viable for at least 22 days when stored at room temperature. Predictive modeling showed that such probiotics remain substantially viable despite periodic short-term spikes in storage temperature that can exceed room temperature by up to 60°C.

Generally, probiotics are administered to the animal in amounts of from about one to about twenty billion colony forming units (CFUs) per day for the healthy maintenance of intestinal microflora, preferably from about 10 million to about 10 billion live bacteria per day. The dietary supplements can be formulated to contain probiotics in the range of about 10⁵ to about 10¹⁰ colony forming units (CFU) per gram of the supplement, although higher concentrations of probiotics can be supplied.

The dietary supplements of the invention can comprise additional substances such as minerals, vitamins, salts, proteins, amino acids, fibers, condiments, colorants, and preservatives. Non-limiting examples of minerals include calcium, phosphorous, potassium, sodium, iron, chloride, boron, copper, zinc, magnesium, manganese, iodine, selenium and the like, and various salts thereof. Non-limiting examples of vitamins include vitamin A, various B vitamins, e.g., niacin, pantothenic acid, folic acid, biotin, vitamin D, and vitamin K. The dietary supplements may also comprise carotenoids such as alpha-carotene, lycopene, lutein, zeaxanthin and beta-cryptoxanthin. Additional ingredients may also be included, for example, inulin, amino acids, and the like. One particularly preferred amino acid is taurine.

The dietary supplements of the invention can further comprise prebiotics. Prebiotics include any substance that alters microflora composition of the gastrointestinal tract by providing a substrate for growth of microorganisms. Prebiotics include, without limitation, natural and synthesized oligosaccharides, soluble fibers, resistant starch, and gums. The oligosaccharides can be linear or branched. The prebiotic can be specifically chosen for its ability to enhance the survival of the probiotic in the storage container, or in the gastrointestinal tract of an animal. The prebiotic can also be specifically chosen for its ability to enhance the functionality of the probiotic in the animal or to complement the benefits of the probiotic. Generally, prebiotics are administered in amounts sufficient to positively stimulate the healthy microflora in the gut and cause these "good" bacteria to reproduce. Typical amounts are from about one to about 10 grams per serving or from about 5 percent to about 40 percent of the recommended daily dietary fiber for an animal.

In various embodiments, the dietary supplements of the invention may further comprise from about 15% to about 60% crude protein. Preferably, the compositions comprise about 40% to about 55% crude protein. The crude protein material may comprise vegetable proteins such as soybean, corn, rice, cottonseed, and peanut, or animal proteins such as casein, albumin, and meat protein. Non-limiting examples of meat protein useful herein include pork, lamb, equine, poultry, fish, and mixtures thereof.

The dietary supplements may further comprise from about 5% to about 40% fat. Preferably, the dietary supplements comprise about 15% to about 22% fat. The dietary supplements may further comprise a source of carbohydrate. The dietary supplements may comprise from about 15% to about 60% carbohydrate. Non-limiting examples of such carbohydrates include grains or cereals such as rice, corn, sorghum, alfalfa, barley, soybeans, canola, oats, wheat, and mixtures thereof. The dietary supplements may also optionally comprise other materials such as dried whey and other dairy by-products.

The dietary supplements may also further comprise at least one fiber source. The dietary supplement may comprise from about 0.5% to about 5% fiber. A variety of soluble or insoluble fibers may be utilized, as will be known to those of ordinary skill in the art. The fiber source can be beet pulp (from sugar beet), gum arabic, gum talha, psyllium, rice bran, carob bean gum, citrus pulp, pectin, fructooligosaccharide, mannanoligofructose, soy fiber, fiber from lupins, arabinogalactan, galactooligosaccharide, arabinoxylan, or mixtures thereof. The fiber source can be a fermentable fiber, as are many of those listed above. Fermentable fiber has previously been described to provide a benefit to the immune system of companion animals. Fermentable fiber or other compositions known to those of skill in the art which provide a prebiotic composition that could enhance the growth of probiotics within the intestine may also be incorporated into the composition to aid in the enhancement of the benefits provided by the present invention to the immune system gastrointestinal system, and general health of an animal.

To enhance the length of time the dietary supplements can be stored, the dietary supplements preferably has a total moisture content between about 2% and about 10% by weight of the supplement. Preferably, the total moisture content is less than 5% by weight of the supplement. Similarly, the dietary supplement preferably has a water activity in the range of 0.20 to 0.6. Preferably, the water activity is less than 0.55.

The dietary supplements can be specially formulated for particular animals such as dogs or cats. Similarly, the dietary supplements may be specially formulated for young, adult, or senior animals. In general, specialized formulations comprise ingredients that meet the energy and nutritional requirements appropriate for particular animals and for particular animals at different stages of development or age, or with specific nutrient requirements related to a disease state.

In one embodiment, the dietary supplement is formulated for dogs. This formula comprises from about 1% to about 90% animal digest, from about 1% to about 30% dried brewers yeast, from about 0.1% to about 10% vitamin C, from about 0.1% to about 10% vitamin E, from about 0.01% to about 3% beta carotene, from about 0.1% to about 5% zinc proteinate, from about 0.01% to about 3% manganese proteinate, from about 0.01% to about 3% ferrous sulfate, from about 0.01% to about 1% copper proteinate, from about 0.01% to about 1% calcium iodate, and from about 0.001% to about 0.1% sodium selenite. In a further embodiment, the dietary supplement further comprises from about 0.1% to about 2% taurine, and from about 1% to about 50% probiotics. The supplement may further comprise from about 0.01% to about 2% sodium chloride.

In another embodiment, the dietary supplement is formulated for cats. This formula comprises from about 0% to about 80% animal digest, from about 0% to about 30% dried brewers yeast, from about 0% to about 10% vitamin C, from about 0% to about 10% vitamin E, from about 0% to about 3% beta carotene, from about 0% to about 5% zinc proteinate, from about 0% to about 2% taurine, from about 0% to about 3% manganese proteinate, from about 0% to about 3% ferrous sulfate, from about 0% to about 1% copper proteinate, from about 0% to about 0.1% calcium iodate, from about 0% to about 0.01% sodium selenite, and from about 1% to about 50% probiotics. The supplement may further comprise from about 0.01% to about 2% sodium chloride.

In the present invention, the probiotics are microencapsulated. The probiotics are microencapsulated within a biocompatible microcapsule to enhance or sustain the viability of the probiotics, particularly during storage. The microcapsule acts as a barrier to protect the probiotics from harmful environmental conditions such as temperature fluctuations, oxygen, moisture, and light. Microcapsules comprise an active agent, e.g., a probiotic core surrounded by a biocompatible shell or coating. Suitable materials for preparation of biocompatible microcapsules and methods for encapsulation are known in the art, see for example, U.S. Pat. Nos. 6,974,592 to Yan, 6,969,530 to Curtis et al., 6,887,493 to Shefer et al. and 6,303,355 to Opara, and U.S. Pat. Application Nos. 10/507,359 to Ubbink et al., and 10/656,386 to Simmons et al. The microcapsules can be prepared for controlled release of the probiotics after administration to an animal. In preferred embodiments, the microcapsules of the invention range from about 0.1mm to about 1.0mm in diameter. In one embodiment, the probiotics are microencapsulated with a biopolymer matrix coated with shellac.

The skilled artisan can determine the appropriate amount of probiotics to be added to a given dietary supplement formulation. Such factors that may be taken into account include the average consumption of specific types of compositions by different animals, whether the animal that is intended to ingest the dietary supplements has any particular health, wellness, or nutritional requirements, or suffers from a particular disease or disorder, the age, sex, size, or breed of the animal, and the manufacturing conditions under which the dietary supplement composition is prepared. The concentrations of probiotics to be added to the supplement can be calculated on the basis of the energy and nutrient requirements of the animal.

The microencapsulated probiotics in the dietary supplement remain substantially viable when the dietary supplement is stored for a period of 11 days at temperatures up to 50°C, 2 days at the temperatures up to 60°C, or 20 hours at temperatures up to 65°C.

In a further aspect, the present invention provides kits suitable for administering a dietary supplement comprising probiotics to an animal. The kits comprise in separate containers in a single package or in separate containers in a virtual package, as appropriate for the kit component, at least one probiotic and at least one of (1) one or more ingredients suitable for consumption by an animal, (2) one or more prebiotics, (3) one or more anti-diarrhea agents, (4) instructions for how to combine the dietary supplement and other kit components, particularly to produce a food composition useful for administering probiotics to an animal, and (5) instructions for how to use the dietary supplement and other components of the present invention, particularly for the benefit of the animal, to maintain or improve gastrointestinal health, and to combine the supplement with food ingredients for consumption by an animal.

When the kit comprises a virtual package, the kit is limited to instructions in a virtual environment in combination with one or more physical kit components. The kit contains the dietary supplement and other components in amounts sufficient to provided beneficial amounts of probiotics to the animal, generally to maintain or improve gastrointestinal health and promote the health and wellness of the animal. Typically, the dietary supplement and the other suitable kit components (e.g., food compositions) are admixed just prior to consumption by an animal. The kits may contain the kit components in any of various combinations and/or mixtures. In one embodiment, the kit contains a packet containing one or more dietary supplement and a container of food for consumption by an animal. The kit may contain additional items such as a device for mixing the dietary supplement and ingredients or a device for containing the admixture, e.g., a food bowl. In another embodiment, the dietary supplement is mixed with additional nutritional supplements such as vitamins and minerals that promote good health in an animal.

The instructions can direct and/or inform a user, pet owner, veterinarian, pet food supplier, and the like that use of the dietary supplement can provide a referenced benefit, for example, the treatment or prevention of diarrhea, the improvement of intestinal health, or the improvement or maintenance of a healthy microflora balance in the gastrointestinal tract of the animal. The instructions can also direct the user how to apply or admix the dietary supplements to the food or water of an animal.

Suitable containers for use with the probiotics and other ingredients of the present invention include, for example, cans, jars, bottles, bottles with shaker lids, mills, vials, syringes, tubes, pouches, sachets, bags, or folders. The containers can be formed from a variety of materials including without limitation glass, plastic, polymers, metals, alloys, metal or alloy foil, rubber, cardboard, or paper. The containers can also comprise a sealant, which can be formed from any material suitable in the art such as a resin or polymer. In a preferred embodiments, the container is a metal or alloy foil pouch such as sachet that comprises a moisture barrier and/or oxygen barrier layers to further enhance the viability of the probiotics when stored at room temperature and in the event of short term temperature spikes above room temperature.

The containers can comprise a moisture barrier and/or oxygen barrier to further enhance the viability of probiotics over long-term storage. Moisture barriers and oxygen barriers are known in the pharmaceutical and food industries. Suitable barriers for use in the present invention are described in U.S. Pat. Nos. 6,716,499 to Vadhar, 6,524,720 to Shah, 5,792,530 to Bonner et al., 5,512,338 to Bianchini et al., and 4,977,004 to Bettle et al. In addition to, or in lieu of such barriers, the containers may comprise an oxygen scavenger and/or a desiccant/moisture absorbing compound. Suitable oxygen scavengers and desiccants are known in the art, for example, U.S. Pat. No. 6,746,622 to Yan et al., 6,387,461 to Ebner et al., and 6,228,284 to Ebner et al. (oxygen scavengers) and U.S. Pat. No. 6,130,263 to Hekal (desiccants).

A label on or associated with the container can indicate useful indications for the dietary supplement. The kit may also optionally comprise additional containers having another supplemental ingredients or agents as described herein. The article of manufacture or kit can comprise instructions for using the dietary supplements to treat various conditions or to support overall health and/or wellness in companion animals such as a dog or a cat. In some embodiments, multiple containers are packaged together as a carton. For example, but not by way of limitation, a carton can comprise 7 containers, equivalent to a one week supply of dietary supplements, or 30 containers equivalent to a one month supply of dietary supplements, and the like. The containers can hold a single dose of the supplement (i.e., single use), or multiple doses of the supplement. The containers can also be packaged with pet food containers, for example, with a can of dog food or cat food, or with dry dog or cat food, or other dietary supplements such as a treat.

In another aspect, the present invention provides a food composition comprising one or more ingredients suitable for consumption by an animal and a dietary supplement of the present invention. The dietary supplement can be admixed with the ingredients to produce the food composition or can be applied to the ingredients to produce the food composition. In one embodiment, the dietary supplement is applied onto a food composition, usually by sprinkling or spraying. In a preferred embodiment, the ingredients form a nutritionally complete companion animal food such as a dog or cat food and, preferably, the dietary supplement is applied onto the food. Typically, the dog or cat food is placed in a suitable container and the dietary supplement is applied onto the food, e.g., sprinkled on the food.

In another aspect, the present invention provides a method for administering a probiotic to an animal comprising administering to the animal a food composition comprising one or more ingredients suitable for consumption by an animal and a dietary supplement of the present invention.

In another aspect, the present invention provides a method for manufacturing a food composition containing probiotics comprising admixing one or more ingredients suitable for consumption by an animal and a dietary supplement of the present invention or applying a dietary supplement of the present invention onto the food composition. In a further aspect, the present invention provides the food compositions manufactured using this method.

In another aspect, the present invention provides a method for promoting the health or wellness in an animal comprising administering to an animal a health or wellness promoting amount of the dietary supplement of the present invention. In one embodiment, the dietary supplement is administered in conjunction with an anti-diarrhea agent.

In another aspect, the present invention provides a method for promoting gastrointestinal health in an animal comprising administering to an animal a gastrointestinal health promoting amount of the dietary supplement of the present invention. In one embodiment, the dietary supplement is administered in conjunction with an anti-diarrhea agent.

For these methods, the supplement can be administered using any suitable method but is preferably administered by admixing the supplement with or applying the supplement onto a food composition, preferably a nutritionally complete food composition.

To promote health and wellness or to promote gastrointestinal health, the probiotics are administered to the animal via the dietary supplement of the present invention in amounts of from about one to about twenty billion colony forming units (CFUs) per day, preferably from about 10 million to about 10 billion live bacteria per day.

In another aspect, the present invention provides a means for communicating information about or instructions for one or more of (1) using the dietary supplement to administer probiotics to an animal, particularly to maintain or improve gastrointestinal health, (2) admixing the dietary supplement with the other components (food compositions) of the present invention, (3) administering the dietary supplement to an animal, alone or in combination with the other elements of the present invention, and (4) using the kits of the present invention to administer probiotics to an animal, particularly to maintain or improve gastrointestinal health or to promote the health or wellness of the animal, e.g., preventing and treating diarrhea or for improving stool quality. The means comprises a document, digital storage media, optical storage media, audio presentation, or visual display containing the information or instructions. In certain embodiments, the communication means is a displayed web site, visual display kiosk, brochure, product label, package insert, advertisement, handout, public announcement, audiotape, videotape, DVD, CD-ROM, computer readable chip, computer readable card, computer readable disk, computer memory, or combination thereof containing such information or instructions. Useful information includes one or more of (1) methods and techniques for combining and administering the dietary supplement and/or other components and (2) contact information for animals or their caregivers to use if they have a question about the invention and its use. Useful instructions include amounts for mixing and administration amounts and frequency. The communication means is useful for instructing on the benefits of using the present invention and communicating the approved methods for administering the invention to an animal.

### EXAMPLES (outside the scope of the invention)

The invention can be further illustrated by the following examples, although it will be understood that these examples are included merely for purposes of illustration and are not intended to limit the scope of the invention unless otherwise specifically indicated.

### Example 1

### Probiotic Powder Blending Process

Probiotic powder formulations for dogs and cats were manufactured using standard dry mixing equipment, including a ribbon mixer, a v-mixer, or double-agitated paddle mixer. Mixing times were variable, depending on the mixer used. The maximal mixing time was 10 minutes. The sequence in which the ingredients of the probiotic composition were added to the mixer was not critical. The blended powder was immediately packaged into bulk bags protected with PVC shroud or into the PVC-lined bags. The formulation prepared for cats is set forth in Table 1 and the formulation for dogs is set forth in Table 2.

**Table 1**

| Probiotic Powder Formulation for Cats | | |
|---|---|---|
| Ingredient | Unit | Amount |
| Carotenes | IU A/lb | 446000 |
| Crude Fiber | % | 0.415 |
| Fat | % | 17.7 |
| Calcium | ppm | 3059 |
| Copper | ppm | 58.12 |
| Iron | ppm | 870.5 |
| Magnesium | ppm | 967.5 |
| Manganese | ppm | 284.8 |
| Phosphorus | ppm | 17690 |
| Potassium | ppm | 7476 |
| Sodium | ppm | 14760 |
| Zinc | ppm | 1144 |
| Moisture | % | 3.39 |
| Protein | % | 46.8 |
| Selenium | ppm | 2.16 |
| Vitamin E | mg/100g | 1110 |
| Vitamin C | ppm | 8670 |
| Taurine | ppm | 6730 |
| Enterococcus faecium | CFU/g | 10⁹ |

**Table 2**

| Probiotic Powder Formulation for Dogs | | |
|---|---|---|
| Ingredient | Unit | Amount |
| Carotenes | IU A/lb | 787000 |
| Crude Fiber | % | 0.560 |
| Fat | % | 17.6 |
| Calcium | ppm | 3721 |
| Copper | ppm | 123.5 |
| Iron | ppm | 1577 |
| Magnesium | ppm | 1001 |
| Manganese | ppm | 685.1 |
| Phosphorus | ppm | 18120 |
| Potassium | ppm | 7506 |
| Sodium | ppm | 17930 |
| Zinc | ppm | 1993 |
| Moisture | % | 3.39 |
| Protein | % | 44.7 |
| Selenium | ppm | 2.81 |
| Vitamin E | mg/100g | 1410 |

| Vitamin C | ppm | 10700 |
|---|---|---|
| Enterococcus faecium | CFU/g | 10⁹ |

### Example 2

### Probiotic Powder Packaging

Approximately 1.15 ± 0.1 grams of blended probiotic powder was packaged into individual single use containers using specialized packaging equipment manufactured by Cloud Packaging Solutions, Des Plains, Il. Containers were constructed as outlined in Table 3. Container size was 3" X 2". The containers comprise of paper, low density polyethylene, foil and Dupont Surlyn®. The WVTR and OTR are given in Table 3.

**Table 3**

| Packaging Specifications | | | |
|---|---|---|---|
| Property | Test Method | Units | Typical Value |
| Thickness | TAPPI T 411 | Mils | 0.003235 |
| Water Vapor Transfer Rate | ASTM F 1249 | g/100 sq. in/24 hr at 37.8°C -100%RH | <0.01 |
| Oxygen Transferred Rate | ASTM 3985 | g/100 sq. in/24 hr at 37.8°C -100%RH | <0.01 |
| Seal Strength | @325°F, 0.5 sec, 40psi (1 jaw heated) | Grams/inch | > 1500 |

### Example 3

### Probiotic Stability In Powder Composition

The dietary supplements include probiotic powder compositions utilizing Enterococcus faecium (SF68) as a representative probiotic microorganism, which was microencapsulated in a biopolymer matrix. Encapsulated compositions were stored at five different temperatures for up to 96 hours. The temperatures were 25°C, 33°C, 45°C, 55°C, and 65°C. Samples from each stored composition were taken at various time points from time 0, as shown in Table 4, and analyzed for viability of the Enterococcus faecium. Results of the viability measurements are summarized in Table 4.

Observed Data and Fitted Arrhenius Model for SF68 in Powder Dietary Supplement. This data shows the observed and predicted average log (counts) of Enterococcus faecium during the short-term storage at four temperatures (25°, 33°C, 45°C, 55°C and 65°C. The Arrhenius model we used is Y = a + (b*hours)*(e(-k/temp)), with coefficients a=9.3348; b=-73214.6 and k=-914.7.

**Table 4**

| Effect of Storage Time and Temperature on Viability of E. faecium During Short-Term Storage | | | | | |
|---|---|---|---|---|---|
| Time | Temperature (°C) | | | | |
| (hr) | 25°C | 33°C | 45°C | 55°C | 65°C |
| 0 | | 9.18 | 9.18 | 9.18 | 9.18 |
| 8 | | | 9.30 | 9.08 | 9.23 |
| 10 | | | | | 8.70 |
| 12 | | | | | 9.15 |
| 14 | | | | | 8.95 |
| 16 | | | 9.48 | 9.30 | 9.20 |
| 18 | | | | | 8.97 |
| 20 | | | | | 8.63 |
| 24 | 9.20 | 9.46 | | | 7.83 |
| 26 | | | | | 7.26 |
| 28 | | | | | 7.11 |
| 30 | | | | | 6.54 |
| 32 | | | 9.40 | 9.38 | 8.11 |
| 40 | | | 8.89 | 8.83 | 7.30 |
| 48 | 9.30 | 9.48 | | | |
| 56 | | | 9.45 | 9.23 | 5.57 |
| 64 | | | 8.80 | 8.92 | 5.54 |
| 72 | 9.32 | 9.43 | | | |
| 80 | | | 9.70 | 8.94 | 5.00 |
| 88 | | | 9.15 | 9.11 | 4.61 |
| 96 | 9.40 | 9.45 | | | |

Viability of the probiotic organisms in the encapsulated compositions was also evaluated under conditions of sustained high temperatures. Microencapsulated probiotic compositions utilizing E. faecium SF68 were packaged in containers as described in Example 2 and stored for 11 days at45°C, 50°C, 55°C and 60°C. Experiments were carried out in quadruplicates. The results, measured in log colony forming units, are presented in Tables 5. No significant loss of viability was observed at temperature at 50°C or below over the seven day storage period.

**Table 5**

| Effect of Storage Time and Temperature on Viability of E. faecium During Long-Term Storage | | | | |
|---|---|---|---|---|
| | Temperature (°C) | | | |
| Days | 45°C | 50°C | 55°C | 60°C |
| 0 | 9.24 | 9.24 | 9.24 | 9.24 |
| 1 | | 9.20 | 9.27 | 9.21 |
| 2 | | 9.23 | 9.21 | 8.29 |
| 3 | | 9.11 | 9.16 | 6.81 |
| 4 | | 9.23 | 9.20 | 5.04 |
| 5 | 9.17 | 9.17 | 8.92 | 4.75 |
| 6 | 8.97 | 8.95 | 8.70 | 4.40 |
| 7 | 8.95 | 8.92 | 8.63 | 3.79 |
| 8 | 8.96 | 8.83 | 8.27 | 3.86 |
| 11 | | 8.74 | 7.16 | |

The results shown in Tables 4 and 5 are in log cfu/g of dietary supplement.

Observed Data and Fitted Arrhenius Model for SF68 in Powder Dietary Supplement. This data shows the observed and predicted average log (counts) of Enterococcus faecium during long-term storage at four temperatures (45°, 50°C, 55°C and 60°C. The Arrhenius model we used is Y = a + (b*days)*(e(-k/temp)), with coefficients a=9.2249; b=-3.0488 and k=-1187.4.

### Example 4

### Probiotic Powder Composition Acceptance in Dogs and Cats

Probiotic powder compositions were specially formulated for dogs and for cats. The compositions were administered by means of topically sprinkling 1.0 grams of the composition onto the animals' dry extruded diet, or by homogenously mixing the 1.0 grams of the composition with the dry extruded diet.

Three groups of twenty dogs were evaluated on consecutive days. On the first day Group 1 measured preference for the topically applied probiotic composition relative to control, Group 2 measured preference for the homogenously mixed probiotic composition relative to control and Group 3 measured preference for the topically applied probiotic composition relative to the homogenously mixed probiotic composition. On the second day the diet placement was reversed. Within each group, the dogs were provided with two possible food choices at feeding time each containing 700 grams of diet without supplementation (control), Supplementation was added, as outlined in feeding protocol, before feeding the dogs. Each bowl of food was weighed prior to the consumption period. Animals were given twenty minutes to eat. Preference was determined by weighing each bowl of food (control and supplemented) after the 20-minute consumption period.

Three groups of twenty-seven cats were evaluated on consecutive days. On the first day Group 1 measured preference for the topically applied probiotic composition relative to control, Group 2 measured preference for the homogenously mixed probiotic composition relative to control and Group 3 measured preference for the topically applied probiotic composition relative to the homogenously mixed probiotic composition. On the second day the diet placement was reversed. Within each group, the cats were provided with two possible food choices at feeding time each containing approx 100 grams of diet without supplementation (control), Supplementation was added, as outlined in feeding protocol, before feeding the cats. Each bowl of food was weighed prior to the consumption period. Animals were given 18 hours to eat. Preference was determined by recording reduction in weight of each diet on an automatic scale.

In summary, diets containing the probiotic preparation (either sprinkled or mixed with the diets) were significantly preferred by cats or were on parity or significantly preferred by dogs.

In the specification, there have been disclosed typical preferred embodiments of the invention and, although specific terms are employed, they are used in a generic and descriptive sense only and not for purposes of limitation, the scope of the invention being set forth in the claims. Obviously many modifications and variations of the invention are possible in light of the above teachings. It is therefore to be understood that within the scope of the appended claims the invention may be practiced otherwise than as specifically described.

## Claims

1. A dietary supplement comprising at least one microencapsulated probiotic and animal digest, dried brewers yeast, vitamin C; vitamin E, beta carotene, zinc proteinate, manganese proteinate, ferrous sulfate, copper proteinate, calcium iodate, and sodium selenite.

2. The supplement of claim 1 wherein the probiotic comprises (i) a prokaryote, eukaryote, or archaebacteria probiotic; or (ii) at least one of any suitable strain or subspecies of Enterococcus, Streptococcus, Lactobacillus, Lactococcus, Bifidobacterium, or Saccharomyces; or (iii) Enterococcus faecium (SF68).

3. The supplement of claim 1 further comprising taurine or a prebiotic or an anti-diarrhea agent.

4. The supplement of claim 1 wherein the microcapsule comprises a biopolymer matrix coated with shellac.

5. The dietary supplement of claim 1 wherein the dietary supplement is formulated for a companion animal; optionally wherein the companion animal is a cat or dog.

6. A food composition comprising one or more ingredients suitable for consumption by an animal and a dietary supplement of claim 1; wherein (i) the ingredients form a nutritionally complete companion animal food, (ii) the dietary supplement is admixed with the ingredients; or (iii) the dietary supplement is applied to the ingredients.

7. A method for administering a probiotic to an animal comprising administering to the animal a food composition comprising one or more ingredients suitable for consumption by an animal and a dietary supplement of claim 1.

8. A method for manufacturing a food composition containing probiotics comprising admixing one or more ingredients suitable for consumption by an animal and a dietary supplement of claim 1 or applying a dietary supplement of claim 1 onto the food composition.

9. The dietary supplement of claim 1 for use in promoting the health or wellness in an animal: optionally wherein (i) the dietary supplement is administered by admixing the supplement with or applying the supplement onto a food composition comprising one or more ingredients suitable for consumption by an animal; or (ii) the dietary supplement is administered in conjunction with one or more anti-diarrhea agents.

10. The dietary supplement of claim 1 for use in promoting the gastrointestinal health in an animal; optionally wherein (i) the dietary supplement is administered by admixing the supplement with or applying the supplement onto a food composition comprising one or more ingredients suitable for consumption by an animal; or (ii) the dietary supplement is administered in conjunction with one or more anti-diarrhea agents.

## Patentansprüche

1. Nahrungsergänzungsmittel, umfassend mindestens ein mikroverkapseltes probiotisches und tierisches Aufschlussprodukt, getrocknete Brauhefe, Vitamin C; Vitamin E, Betacarotin, Zinkproteinat, Manganproteinat, Eisensulfat, Kupferproteinat, Calciumiodat und Natriumselenit.

2. Ergänzungsmittel nach Anspruch 1, wobei das Probiotikum (i) ein prokaryotisches, eukaryotisches oder Archeenprobiotikum; oder (ii) mindestens eines von einem beliebigen geeigneten Stamm oder einer beliebigen geeigneten Subspezies von Enterococcus, Streptococcus, Lactobacillus, Lactococcus, Bifidobacterium oder Saccharomyces; oder (iii) Enterococcus faecium (SF68) umfasst.

3. Ergänzungsmittel nach Anspruch 1, ferner umfassend Taurin oder ein Präbiotikum oder ein Antidiarrhoe-Mittel.

4. Ergänzungsmittel nach Anspruch 1, wobei die Mikrokapsel eine Biopolymermatrix umfasst, die mit Schellack beschichtet ist.

5. Nahrungsergänzungsmittel nach Anspruch 1, wobei das Nahrungsergänzungsmittel für ein Begleittier formuliert ist; wobei gegebenenfalls das Begleittier eine Katze oder ein Hund ist.

6. Nahrungsmittelzusammensetzung, umfassend einen oder mehrere Inhaltsstoffe, die für den Verzehr durch ein Tier geeignet sind, und ein Nahrungsergänzungsmittel nach Anspruch 1; wobei (i) die Inhaltsstoffe ein ernährungsmäßig vollständiges Haustierfutter bilden, (ii) das Nahrungsergänzungsmittel den Inhaltsstoffen beigemischt ist; oder (iii) das Nahrungsergänzungsmittel auf die Inhaltsstoffe aufgebracht ist.

7. Verfahren zum Verabreichen eines Probiotikums an ein Tier, umfassend das Verabreichen einer Nahrungsmittelzusammensetzung an das Tier, umfassend einen oder mehrere Inhaltsstoffe, die für den Verzehr durch ein Tier geeignet sind, und ein Nahrungsergänzungsmittel nach Anspruch 1.

8. Verfahren zum Herstellen einer Probiotika enthaltenden Nahrungsmittelzusammensetzung, umfassend das Beimischen eines oder mehrerer Inhaltsstoffe, die für den Verzehr durch ein Tier geeignet sind, und eines Nahrungsergänzungsmittels nach Anspruch 1, oder das Aufbringen eines Nahrungsergänzungsmittels nach Anspruch 1 auf die Nahrungsmittelzusammensetzung.

9. Nahrungsergänzungsmittel nach Anspruch 1 zum Verwenden bei der Förderung der Gesundheit oder des Wohlergehens bei einem Tier: wobei gegebenenfalls (i) das Nahrungsergänzungsmittel verabreicht wird durch Beimischen des Ergänzungsmittels mit einer oder Aufbringen des Ergänzungsmittels auf eine Nahrungsmittelzusammensetzung, die einen oder mehrere Inhaltsstoffe umfasst, die für den Verzehr durch ein Tier geeignet sind; oder (ii) das Nahrungsergänzungsmittel in Verbindung mit einem oder mehreren Antidiarrhoe-Mitteln verabreicht wird.

10. Nahrungsergänzungsmittel nach Anspruch 1 zum Verwenden bei der Förderung der Magen-Darm-Gesundheit bei einem Tier: wobei gegebenenfalls (i) das Nahrungsergänzungsmittel verabreicht wird durch Beimischen des Ergänzungsmittels mit einer oder Aufbringen des Ergänzungsmittels auf eine Nahrungsmittelzusammensetzung, die einen oder mehrere Inhaltsstoffe umfasst, die für den Verzehr durch ein Tier geeignet sind; oder (ii) das Nahrungsergänzungsmittel in Verbindung mit einem oder mehreren Antidiarrhoe-Mitteln verabreicht wird.

## Revendications

1. Complément diététique comprenant au moins un probiotique et un digestat animal micro-encapsulés, des levures de bière séchées, de la vitamine C ; de la vitamine E, du bêta-carotène, du protéinate de zinc, du protéinate de manganèse, du sulfate ferreux, du protéinate de cuivre, de l'iodate de calcium et du sélénite de sodium.

2. Complément selon la revendication 1, dans lequel le probiotique comprend (i) un probiotique de procaryote, d'eucaryote ou d'archéobactéries ; ou (ii) au moins l'une parmi n'importe quelle souche ou sous-espèce appropriée d'Enterococcus, Streptococcus, Lactobacillus, Lactococcus, Bifidobacterium ou Saccharomyces ; ou (iii) Enterococcus faecium (SF68).

3. Complément selon la revendication 1, comprenant en outre de la taurine ou un prébiotique ou un agent antidiarrhéique.

4. Complément selon la revendication 1, dans lequel la microcapsule comprend une matrice biopolymère revêtue de shellac.

5. Complément diététique selon la revendication 1, où le complément diététique est formulé pour un animal de compagnie ; éventuellement dans lequel l'animal de compagnie est un chat ou un chien.

6. Composition alimentaire comprenant un ou plusieurs ingrédients appropriés pour une consommation par un animal et un complément diététique selon la revendication 1 ; dans laquelle (i) les ingrédients forment une nourriture pour animal de compagnie complète sur le plan nutritionnel, (ii) le complément diététique est mélangé aux ingrédients ; ou (iii) le complément diététique est appliqué aux ingrédients.

7. Procédé pour administrer un probiotique à un animal comprenant l'administration à l'animal d'une composition alimentaire comprenant un ou plusieurs ingrédients appropriés pour une consommation par un animal et un complément diététique selon la revendication 1.

8. Procédé de fabrication d'une composition alimentaire contenant des probiotiques, comprenant le mélange d'un ou plusieurs ingrédients appropriés pour une consommation par un animal et d'un complément diététique selon la revendication 1 ou l'application d'un complément diététique selon la revendication 1 sur la composition alimentaire.

9. Complément diététique selon la revendication 1, destiné à être utilisé pour promouvoir la santé ou le bien-être chez un animal : éventuellement dans lequel (i) le complément diététique est administré en mélangeant le complément avec, ou en appliquant le complément sur, une composition alimentaire comprenant un ou plusieurs ingrédients appropriés pour une consommation par un animal ; ou (ii) le complément diététique est administré conjointement avec un ou plusieurs agents antidiarrhéiques.

10. Complément diététique selon la revendication 1, destiné à être utilisé pour promouvoir la santé gastro-intestinale chez un animal ; éventuellement dans lequel (i) le complément diététique est administré par mélange du complément avec, ou application du complément sur, une composition alimentaire comprenant un ou plusieurs ingrédients appropriés pour une consommation par un animal ; ou (ii) le complément diététique est administré conjointement avec un ou plusieurs agents antidiarrhéiques.
